(19) Europäisches Patentamt
European Patent Office
Office européen des brevets

(11) EP 0 600 359 B1

(12) EUROPEAN PATENT SPECIFICATION

(45) Date of publication and mention of the grant of the patent:
**23.07.1997 Bulletin 1997/30**

(51) Int Cl.[6]: **C11D 3/39**, D06L 3/02

(21) Application number: **93118885.8**

(22) Date of filing: **24.11.1993**

# (54) Process for increasing the bleaching efficiency of an inorganic persalt or of hydrogen peroxide

Verfahren zum Erhöhen der Bleichwirksamkeit eines anorganischen Persalts oder Wasserstoffperoxids

Procédé pour augmenter l'efficacité de blanchiment d'un persel ou de l'eau oxygénée

(84) Designated Contracting States:
**AT BE CH DE DK ES FR GB IE IT LI NL PT SE**

(30) Priority: **02.12.1992 IT MI922759**

(43) Date of publication of application:
**08.06.1994 Bulletin 1994/23**

(73) Proprietor: **AUSIMONT S.p.A.**
**I-20121 Milano (IT)**

(72) Inventors:
- **Burzio, Fulvio**
  **I-20145 Milano (IT)**
- **Beck, Roland**
  **B-1160 Brussels (BE)**
- **Lubineau, André**
  **F-91410 Dourdan (FR)**
- **Augé, Jacques**
  **F-91190 Gif-sur Yvette (FR)**
- **Drouillat, Bruno**
  **F-75012 Paris (FR)**
- **Mentech, Julio**
  **F-69100 Villeurbanne (FR)**

(74) Representative: **Sama, Daniele, Dr. et al**
**Sama Patents**
**Via Morgagni, 2**
**20129 Milano (IT)**

(56) References cited:
**WO-A-92/06155** **WO-A-92/16493**

- **JOURNAL OF THE AMERICAN CHEMICAL SOCIETY, vol. 62, November 1940 US, pages 2960-2961, C. NIEMANN, J. HAYS 'The Structure of N-Acetyl-d-glucosylamine'**
- **JOURNAL OF ORGANIC CHEMISTRY, vol. 46, 1981 US, pages 3158-3160, J. KIRK SMITH ET AL 'Synthesis of N-Glycosides'**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 600 359 B1

## Description

The invention relates to a process for increasing the bleaching efficiency of an inorganic persalt or of hydrogen peroxide, or of bleaching and/or detergent compositions suitable for removing stains from textiles or from other products such as paper, cellulose, cork, hair, etc.

The inorganic persalts to which the process of the present invention can be applied are substances capable of releasing hydrogen peroxide in an aqueous solution. Examples of such inorganic persalts are alkali metal perborates, percarbonates, persulfates, persilicates, perpyrophosphates, and their mixtures. Preferred alkali metals are Na, K and Li, particularly Na. Among the inorganic persalts, sodium perborate monohydrate, sodium perborate tetrahydrate or mixtures thereof are the most commonly employed.

When an inorganic persalt is used alone, it provides a satisfactory bleaching effect at the boil, but at a lower temperature, such as at 40° C or 60°C, where the washing machines operate, its bleaching efficiency is extremely low.

Due to the trend of lowering the washing temperature, both for energy saving reasons and as a consequence of the diffused use of synthetic and coloured fabrics, bleaching activators have been developed which improve the bleaching efficiency of inorganic persalts.

US patent No. 2955905 describes a bleaching composition containing an inorganic persalt associated with an organic carboxylic ester which acts as a bleaching activator.

Examples of esters are, among others, esters of polyhydric aliphatic alcohols, such as hexaacetyl mannitol, hexaacetyl sorbitol, and esters of mono- and disaccharides, such as pentaacetyl fructose, pentaacetyl glucose, tetraacetyl glucose and octaacetyl sucrose.

It is not possible to predict which specific acetylated carbohydrates or acetylated polyols are suitable to improve the bleaching efficiency of inorganic persalts or of hydrogen peroxide. In fact only few of them are efficacious.

The Applicant has now surprisingly found that acetylated glucosylamines show a very high activity as bleaching activators. Therefore, acetylated glucosylamines form a new class of bleaching activators.

Thus, an object of the present invention is a process for increasing the bleaching efficiency of an inorganic persalt or of hydrogen peroxide, wherein an acetylated glucosylamine, or a mixture of different acetylated glucosylamines, is added to said inorganic persalt or hydrogen peroxide.

Acetylated glucosylamines are well known in the art. J.Am.Chem.Soc. 62, 2960-1 (1940) describes the structure of N-Acetyl-d-glucosylamine and method to prepare it from a glucoseammonia condensation product, and pentaacetyl-d-glucosylamine from N-acetyl-d-glucosylamine.

J.Org.Chem. 46, 3158-60 (1981) reports the synthesis of N-acetyl-2,3,4,6-tetra-O-acetyl-β-D-glucopyranosylamine from 2,3,4,6-tetra-O-benzyl with acetonitrile in the presence of trifluoromethanesulfonic anhydride.

The glucosylamine has formula:

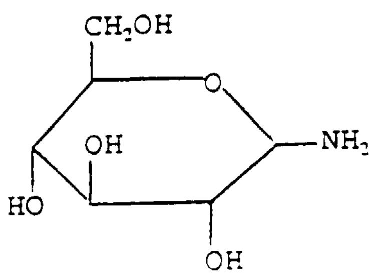

(I)

The average O-acetylation degree of said acetylated glucosylamine is from 1 to 4, preferably from 3 to 4 and most preferably is 4, whereas the average N-acetylation degree is from 1 to 2 and most preferably is 1. The total average acetylation degree is from 2 to 6, preferably from 4 to 6 and most preferably is 5. A particularly preferred acetylated glucosylamine is N-acetyl-tetraacetyl-glucosylamine.

The acetylated glucosylamines according to the invention, hereinafter referred to as AGAs, can be prepared, according to methods well known in the art, by direct acetylation of the glucosylamine having formula (I). The glucosylamine can be prepared, according to known methods, by direct amination of glucose.

The AGAs according to the present invention show, contrary to other acetylated carbohydrate derivatives, an efficacy as bleaching activators even higher than the best activating agents known so far, for example TAED (tetraacetylethylenediamine). The AGAs according to the present invention show the advantage with respect to TAED that they are preparable from natural, renewable products and not from substances directly or indirectly derived from oil. Therefore, the use of AGAs contributes to the protection of the environment.

The higher the amount of added AGA (up to the stoichiometric ratio with the persalt or hydrogen peroxide), the

higher the efficiency of the bleaching agent. Such property, common with TAED, makes AGAs particularly suitable for satisfying the requirement of using higher amount of activating agents in the bleaching and/or detergent compositions, aiming to an increased bleaching efficency.

AGAs may be added as such to the composition containing the persalt, or they can be previously admixed with the persalt. The bleaching compositions or detergent formulations containing AGA and persalt may also contain other conventional components, such as anionic, non-ionic or amphoteric surfactants, alkali metal salts (e.g. sodium carbonate, sodium tripolyphosphate), neutral salts (e.g. sodium sulphate), zeolites, carboxymethyl cellulose, perfumes, enzymes, etc.

The molar ratio between the bleaching activator and the persalt or hydrogen peroxide usually is from 10:90 to 50: 50. When the persalt or the hydrogen peroxide is utilized at low temperatures and for short times, the molar ratio of AGA to persalt or hydrogen peroxide is preferably further increased.

The following examples are provided for merely illustrative purpose and do not limit in any way the scope of the invention.

<u>Preparation of AGA</u>

An AGA having the structure corresponding to N-acetyltetraacetyl-glucosylamine was prepared as follows (see H. S. Isbell, H.L. Frush, "Methods of carbohydrate chemistry" - 1980, VIII, 225-259).

A. <u>Preparation of glucosylamine</u>

20 g of glucose and 0.5 g of ammonium chloride were added to 50 ml of methanol.

The mixture, cooled at 0°C, was treated with ammonia gas until glucose dissolved. Then the solution was stored at 0°C until satisfactory crystallization of glucosylamine occured. The first crystallization may be slow and may require a storage time as long as one month. However, with the aid of seed crystals, which act as aggregation nuclei, the period of crystallization was shortened to a few days.

The crystals were separated and washed with methanol. Then the crude product was dissolved in an equal amount of water and the solution was diluted with an approximately ten-fold volume of methanol, followed by sufficient ethanol to produce incipient turbidity. After about two days, the crystals were separated, washed with methanol and stored over sodium hydroxide in an atmosphere of ammonia.

B. <u>Acetylation of glucosylamine</u>

The glucosylamine prepared according to the procedure described above was acetylated at 0°C using a mixture of acetic anhydride and pyridine. In details, 1 g of glucosylamine was dissolved in 13.6 ml of pyridine. Then, under mild stirring, 9.5 ml of acetic anhydride were added at 0°C.

The solution was kept at rest overnight at room temperature and then poured into 100 ml of HCl 0.1 M. The acetylated glucosylamine was extracted by means of 100 ml of dichloromethane. After neutralization with 100 ml of a saturated aqueous solution of sodium bicarbonate, dichloromethane was completely evaporated and the residue recrystallized from ethanol.

**<u>EXAMPLE 1</u>**

An automatic washing machine (IGNIS®) was run under the following conditions:

- washing program at 60°C;
- load: 3 kg of cotton swatch (white and clean) per washing cycle;
- 6 g/washing cycle of AGA prepared as described above (corresponding to 4% bleaching activator in the detergent), were added to the following detergent composition (which was also used in all the other examples):
  - sodium perborate tetrahydrate: 30 g/washing
  - detergent base (phosphorous-free, free of bleaching agents) 114 g/washing

Said detergent base contained:

| | (%) w/w |
|---|---|
| - overall surfactants (linear sodium dodecylbenzene sulphonate + soap + $C_{13}$-$C_{15}$ alcohol, ethoxylated with 7 EO) | 15.4 |
| - zeolite (4Å) | 28.6 |
| - sodium silicate ($SiO_2/Na_2O$=2) | 4.4 |
| - sodium carbonate | 16.5 |
| - sodium sulfate | 26.5 |
| - carboxymethyl cellulose | 1.2 |
| - antiincrustation copolymers | 4.8 |
| - optical bleaching agents | 0.3 |
| - water up to | 100.0 |

For the determination of the bleaching efficiency (bleaching booster activity) the clean linen was washed together with 2 swatches per washing cycle, previously stained in a standard way, with red wine, at the European Institute of Sankt Gallen (Switzerland) (EMPA 114). At the end of each washing cycle said 2 samples were dried and ironed; the whiteness degree was then measured by means of an Elrepho-Zeiss reflectometer. The resulting bleaching percentage (measure of the bleaching efficiency), reported in Table 1, was determined by the formula:

$$\text{bleaching (\%)} = (A-B)/(C-B) \times 100$$

where:

A = whiteness degree of the sample after the washing;
B = whiteness degree of the sample before the washing;
C = whiteness degree of the sample completely bleached.

The whiteness degree of the swatches is expressed as percentage with respect to MgO, used as a standard, carrying out the measurements with a filter N.6 (wavelength = 464 nm). The thus obtained percentage (74%) is reported in Table 1, along with the results of the other examples.

**EXAMPLES 2-3**

Example 1 was repeated by increasing the amount of activator AGA to 12 and 18 g/washing cycle, corresponding to 8 and 12% in the detergent, respectively.

The obtained results are reported in Table 1.

**EXAMPLES 4-6**

Examples 1-3 were repeated with the difference that the washing temperature was 40°C instead of 60°C.

The obtained results are reported in Table 1.

**EXAMPLES 7-24** (comparative examples)

Examples 1-6 were repeated with the difference that TAED or octaacetyl sucrose or hexaacetyl sorbitol was used instead of AGA. The obtained results are reported in Table 1.

**EXAMPLE 25** (comparative example)

Example 1 was repeated with the difference that sodium perborate tetrahydrate was used alone in the absence of any bleaching activator. The obtained result is reported in Table 1.

**EXAMPLE 26** (comparative example)

Example 4 was repeated with the difference that sodium perborate tetrahydrate was used alone, in the absence

of any bleaching activator. The obtained result is reported in Table 1.

TABLE 1

| ACTIVATOR | | ACTIVATOR AMOUNT (g/washing cycle) | | |
|---|---|---|---|---|
| | | 6 | 12 | 18 |
| | | BLEACHING % | | |
| AGA | 60°C | 74 | 79 | 83 |
| | 40°C | 53 | 56 | 57 |
| TAED (*) | 60°C | 69 | 75 | 82 |
| | 40°C | 51 | 54 | 56 |
| OCTAACETYL SUCROSE (*) | 60°C | 64 | 65 | 57 |
| | 40°C | 44 | 45 | 46 |
| HEXAACETYL SORBITOL (*) | 60°C | 64 | 66 | 68 |
| | 40°C | 45 | 47 | 48 |
| NO ACTIVATOR (*) | 60°C | 55 | | |
| | 40°C | 40 | | |

(*) Comparative tests.

## Claims

1. A process for increasing the bleaching efficiency of an inorganic persalt or of hydrogen peroxide, wherein as activating agent an acetylated glucosylamine having average O-acetylation degree from 1 to 4, and average N-acetylation degree from 1 to 2, or a mixture thereof, is added to said inorganic persalt or hydrogen peroxide.

2. The process according to claim 1, wherein the average O-acetylation degree is from 3 to 4.

3. The process according to claim 2, wherein the average O-acetylation degree is 4.

4. The process according to claim 1, wherein the average N-acetylation degree is 1.

5. The process according to claim 1, wherein the acetylated glucosylamine is N-acetyl-tetraacetyl-glucosylamine.

6. The process according to anyone of the preceding claims, wherein the molar ratio between the acetylated glucosylamine and the inorganic persalt or hydrogen peroxide is from 10:90 to 50:50.

7. The process according to anyone of the preceding claims, wherein the inorganic persalt is selected from sodium perborate monohydrate, sodium perborate tetrahydrate and mixtures thereof.

8. Bleaching and/or detergent compositions comprising an inorganic persalt or hydrogen peroxide and an acetylated glucosylamine as defined in claims 1 to 7.

## Patentansprüche

1. Verfahren zur Erhöhung der Bleicheffizienz eines anorganischen Persalzes oder von Wasserstoffperoxid, in welchem als Aktivierungsmittel ein acetyliertes Glucosylamin mit einem durchschnittlichen O-Acetylierungsgrad von 1 bis 4 und einem durchschnittlichen N-Acetylierungsgrad von 1 bis 2 oder einer Mischung davon dem anorganischen Persalz oder Wasserstoffperoxid zugesetzt wird.

2. Verfahren nach Anspruch 1, in welchem der durchschnittliche O-Acetylierungsgrad 3 bis 4 beträgt.

3. Verfahren nach Anspruch 2, in welchem der durchschnittliche O-Acetylierungsgrad 4 beträgt.

4. Verfahren nach Anspruch 1, in welchem der durchschnittliche N-Acetylierungsgrad 1 beträgt.

5. Verfahren nach Anspruch 1, in welchem das acetylierte Glucosylamin N-Acetyltetraacetylglucosylamin ist.

6. Verfahren nach irgendeinem der vorangehenden Ansprüche, in welchem das Molverhältnis zwischen dem acetylierten Glucosylamin und dem anorganischen Persalz oder Wasserstoffperoxid 10:90 bis 50:50 beträgt.

7. Verfahren nach irgendeinem der vorangehenden Ansprüche, in welchem das anorganische Persalz aus Natriumperborat-monohydrat, Natriumperborat-tetrahydrat und Mischungen derselben ausgewählt wird.

8. Bleich- und/oder Detergens-Zusammensetzungen, umfassend ein anorganisches Persalz oder Wasserstoffperoxid und ein acetyliertes Glucosylamin wie in den Ansprüchen 1 bis 7 definiert.

## Revendications

1. Procédé pour augmenter l'efficacité de blanchiment d'un persel minéral ou du peroxyde d'hydrogène, dans lequel, comme agent d'activation, une glucosylamine acétylée ayant un degré d'O-acétylation moyen de 1 à 4 et un degré de N-acétylation moyen de 1 à 2, ou un mélange de telles glucosylamines acétylées, est ajouté audit persel minéral ou peroxyde d'hydrogène.

2. Procédé selon la revendication 1, dans lequel le degré d'O-acétylation moyen est de 3 à 4.

3. Procédé selon la revendication 2, dans lequel le degré d'O-acétylation moyen est de 4.

4. Procédé selon la revendication 1, dans lequel le degré de N-acétylation moyen est de 1.

5. Procédé selon la revendication 1, dans lequel la glucosylamine acétylée est la N-acétyl-tétraacétylglucosylamine.

6. Procédé selon l'une quelconque des revendications précédentes, dans lequel le rapport molaire entre la glucosylamine acétylée et le persel minéral ou le peroxyde d'hydrogène est de 10:90 à 50:50.

7. Procédé selon l'une quelconque des revendications précédentes, dans lequel le persel minéral est choisi parmi le perborate de sodium monohydraté, le perborate de sodium tétrahydraté et leurs mélanges.

8. Compositions de blanchiment et/ou détergentes comprenant un persel minéral ou du peroxyde d'hydrogène et une glucosylamine acétylée telle que définie à l'une quelconque des revendications 1 à 7.